# EUROPEAN PATENT SPECIFICATION

(11) **EP 3 097 862 B1**
(45) Date of publication and mention of the grant of the patent: **21.08.2019**
(21) Application number: 15737324.2
(22) Date of filing: 16.01.2015
(51) Int. Cl.: A61B 10/00, A61B 5/055

(54) **MRI INDEX ESTIMATION METHOD, AND BIOMETRIC DEVICE**
MRT-INDEX-SCHÄTZVERFAHREN UND BIOMETRISCHE VORRICHTUNG
PROCÉDÉ D'ESTIMATION D'INDICE IRM, ET DISPOSITIF BIOMÉTRIQUE

(30) Priority: 20.01.2014 JP 2014007509
(43) Date of publication of application: 30.11.2016
(73) Proprietor: Kurume University, Kurume-shi Fukuoka 830-0011 (JP); Hamamatsu Photonics K.K., Hamamatsu-shi, Shizuoka 435-8558 (JP)
(72) Inventor: IWATA, Osuke, Kurume-shi Fukuoka 830-0011 (JP); IWATA, Sachiko, Kurume-shi Fukuoka 830-0011 (JP); KURATA, Tsuyoshi, Kurume-shi Fukuoka 830-0027 (JP); YAMAKI, Etsuko, Hamamatsu-shi Shizuoka 435-8558 (JP); ODA, Motoki, deceased (JP)
(74) Representative: Grünecker Patent- und Rechtsanwälte PartG mbB
(86) International application number: PCT/JP2015/051123
(87) International publication number: WO 2015/108158

(56) References cited:
- JP-A- H07 239 299
- JP-A- 2003 093 390
- JP-A- 2010 178 799
- JP-A- 2012 085 965
- OSUKE IWATA ET AL.: 'MR Teiryo Gazo' SHUSANKI NO GAZO SHINDAN vol. 43, no. 2ND ED, 12 December 2013, pages 666 - 671, XP008184742

## Description

### Technical Field

The present invention relates to an MRI index estimation method and a biometric device.

### Background Art

Patent Literature 1 describes a biological-information measuring device using near-infrared spectroscopy. This device automatically calculates a signal related to functional MRI (fMRI) based on a signal value acquired from magnetic resonance imaging (MRI) and near-infrared rays in a calculating unit of a computer equipped in a brain function measuring device using MRI and near-infrared rays.

Patent Literature 2 describes an optical subcutaneous fat thickness measuring device. This device includes a light emitting element, a first light receiving element, and a second light receiving element. The device also includes a database unit storing a non-linear function shared for each region of a living body. The non-linear function correlates a ratio between received light intensities acquired from the first and second light receiving elements and a value of the subcutaneous fat thickness in each region of a living body. This device refers to the non-linear function based on the ratio between received light intensities and estimates the value of the subcutaneous fat thickness.

Non Patent Literature 1 describes a relationship between gestational age and scattering coefficient of forehead of infant obtained by time-resolved spectroscopy (TRS) that is one of near-infrared spectroscopy.

JP H07 239299 A relates to a data processing method of an optical ct apparatus and JP 2012 085965 A to a mammographic measuring apparatus. Osuke Iwata et al relates to an "MR fixed-quantity image", Perinatal Image diagnosis (special extra issue), vol. 43, no. 2nd Ed, pages 666 - 671, XP008184742 (12 December 2013).

### Citation List

### Patent Literature

Patent Literature 1: Japanese Unexamined Patent Publication No. 2003-93390
Patent Literature 2: Japanese Unexamined Patent Publication No. 2010-178799

### Non Patent Literature

Non Patent Literature 1: Ijichi et al., "Developmental changes of optical properties in neonates determined by near-infrared time-resolved spectroscopy", Pediatric Research, 58(3), p.568, 2005

### Summary of Invention

### Technical Problem

Now, imaging a measurement site of an examinee using an MRI device allows acquisition of various MRI indexes such as, for example, apparent diffusion coefficient (ADC) and Fractional Anisotopy (FA). However, the measurement using an MRI device results in a high cost and needs remission, making it difficult to repeat the measurement in a short cycle. For instance, an MRI device is currently used to measure a cephalic part of a high-risk infant, but the measurement is performed only at the time of hospital discharge, and frequent measurement is difficult.

The present invention is conceived in light of such problems, and an object thereof is to provide an estimation method and a biometric device capable of acquiring an MRI index of a measurement site by a simple method and device as compared with MRI.

### Solution to Problem

In order to solve the above issue, an MRI index estimation method according to the present invention includes estimating an MRI index based on a scattering coefficient of a measurement site or a parameter having a correlation with the scattering coefficient, the scattering coefficient being obtained by a near-infrared spectroscopy based on a detection result of near-infrared light made incident on the measurement site and propagated inside the measurement site. Furthermore, a biometric device according to the present invention includes: a light incident unit for making near-infrared light incident on a measurement site; a light detection unit for detecting the near-infrared light propagated inside the measurement site; and an estimation calculating unit for obtaining a scattering coefficient of the measurement site or a parameter having a correlation with the scattering coefficient by a near-infrared spectroscopy based on a detection result in the light detection unit, and estimating an MRI index based on the scattering coefficient.

The inventors have found through studies that there exists a significant correlation between scattering coefficient of a measurement site obtained by near-infrared spectroscopy and MRI index. The above estimation method and the biometric device estimates MRI index based on the scattering coefficient of the measurement site obtained by near-infrared spectroscopy or a parameter having a correlation with the scattering coefficient. Non-invasive measurement using near-infrared spectroscopy in this manner allows simple measurement with low cost as compared with MRI, making it possible to, for example, frequently observe a cephalic part of a high-risk infant.

Further, in the above estimation method, the MRI index may be estimated by using a correlative relationship among the scattering coefficient or the parameter, a subarachnoid space thickness, and the MRI index when the MRI index is estimated. In addition, the above biometric device may further include a storage unit for storing a correlative relationship among the scattering coefficient or the parameter, a subarachnoid space thickness, and the MRI index, wherein the estimation calculating unit estimates the MRI index by using the correlative relationship. According to the studies by the present inventors, the relationship between scattering coefficient and MRI index significantly changes depending on subarachnoid space thickness. Therefore, estimating MRI index using correlative relationship among scattering coefficient or the above parameter, subarachnoid space thickness, and MRI index allows estimation of MRI index more precisely.

Further, in the above estimation method and biometric device, the MRI index may be at least one of an ADC and a FA. The inventers have found through studies a very significant correlation between the MRI indexes and the scattering coefficient of the measurement site.

### Advantageous Effects of Invention

The MRI index estimation method and the biometric device according to the invention allow acquisition of MRI index of a measurement site by a simple method and device as compared with MRI.

### Brief Description of Drawings

FIG. 1 is a block diagram schematically illustrating a configuration of a first embodiment of a biometric device according to the invention.
FIG. 2 is a graph illustrating an example of time variations of light intensities of pulsed light emitted from a light incident unit and detection light detected in a light detection unit.
FIG. 3 is a graph illustrating a correlation between ADC and scattering coefficient as an example of a correlation stored in a storage unit.
FIG. 4 is a graph illustrating a correlation between FA and scattering coefficient as another example of the correlation stored in the storage unit.
FIG. 5 is a graph illustrating a correlation between the product of common logarithm value of scattering coefficient and subarachnoid space thickness and ADC as a still another example of the correlation stored in the storage unit.
FIG. 6 is a flowchart illustrating operations of the biometric device and an MRI index estimation method according to the embodiment.
FIG. 7 is a block diagram schematically illustrating a configuration of a second embodiment of the biometric device according to the invention.
FIG. 8 is a graph illustrating a correlation between subarachnoid space thickness and scattering coefficient as an example of the correlation stored in the storage unit.
FIG. 9 is a flowchart illustrating operations of the biometric device and an MRI index estimation method according to the embodiment.
FIG. 10 is a block diagram schematically illustrating a configuration of a biometric device as a third embodiment of the biometric device according to the invention.

### Description of Embodiments

Hereinafter, an embodiment of an MRI index estimation method and a biometric device according to the invention will be described in detail with reference to the accompanying drawings. Note that like reference numbers are used to denote like elements, and thus overlapped description will be omitted.

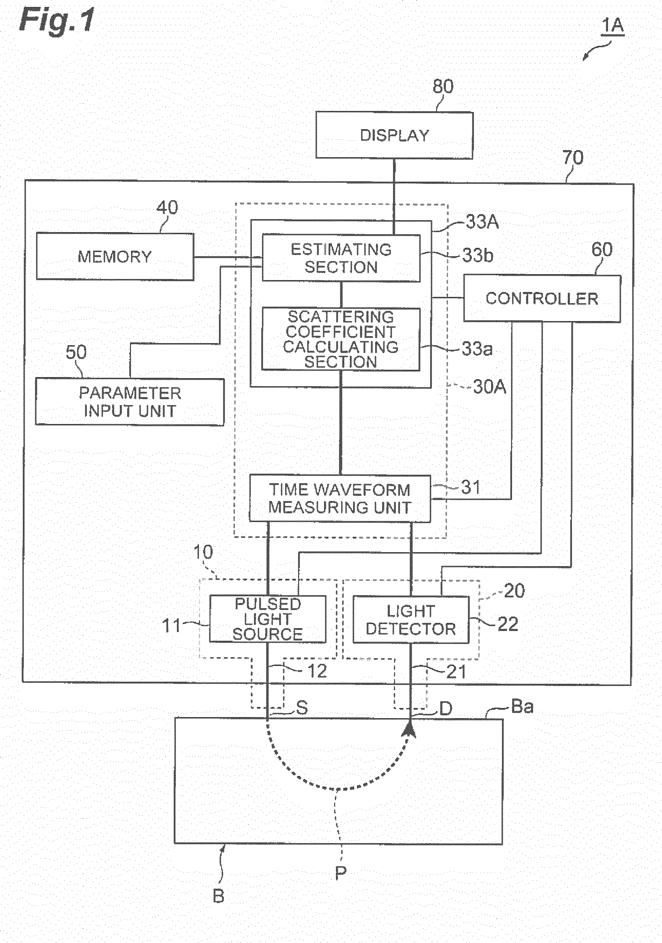

(First Embodiment) FIG. 1 is a block diagram schematically illustrating a configuration of a first embodiment of a biometric device according to the invention. The biometric device 1A is a device that estimates an MRI index (MRI parameter) based on a scattering coefficient of a measurement site B of a living body obtained by time-resolved measurement method using near-infrared light. Herein, the MRI index is a measured quantitative value of a brain organization acquired from a quantitative magnetic resonance map represented by diffusion-weighted image and T1 and T2 relaxation time maps. The MRI index includes, for example, apparent diffusion coefficient (ADC), fractional anisotropy (FA), and T2 relaxation time. Note that T1 is a vertical relaxation time (spin-lattice relaxation time) and T2 is a lateral relaxation time (spin-spin relaxation time). Furthermore, scattering coefficient is a concept including so called reduced scattering coefficient.

The biometric device 1A illustrated in FIG. 1 includes a main body 70 and a display 80. The main body 70 includes a light incident unit 10, a light detection unit 20, an estimation calculating unit 30A, a storage unit 40, a parameter input unit 50, and controller 60 for controlling the light incident unit 10, the light detection unit 20, and the estimation calculating unit 30A.

The light incident unit 10 makes near infrared pulsed light P having a predetermined wavelength incident from a light incident position S of a measurement site B. In the embodiment, one light incident position S is set on a surface Ba of the measurement site B. The light incident unit 10 includes a pulsed light source 11 that generates pulsed light P and a light guide for light incidence 12. An input end of the light guide for light incidence 12 is optically connected with the pulsed light source 11. An output end of the light guide for light incidence 12 is arranged at the light incident position S of the measurement site B.

The pulsed light source 11 to be used includes various devices such as a light emitting diode, a laser diode, and various pulse laser devices. As the pulsed light P to be generated from the pulsed light source 11, near infrared pulsed light is used in which the time width of the pulse is short enough to allow measuring the variation of absorption coefficient of the measurement site B and in which a wavelength having high light absorbing ratio in light absorption properties of the measurement substance is the center wavelength. In an example, a wavelength of the pulsed light P is 760 nm. For example, an optical fiber is used for the light guide for light incidence 12.

The light detection unit 20 detects the pulsed light P propagated inside the measurement site B as detection light. In the embodiment, one light detection position D is set on the surface Ba of the measurement site B. The light detection unit 20 includes a light guide for light detection 21 and a light detector 22 for detecting light to convert the light into an electrical detection signal. An input end of the light guide for light detection 21 is arranged at the light detection position D of the measurement site B. An output end of the light guide for light detection 21 is optically connected with the light detector 22.

The light guide for light detection 21 to be used includes, for example, an optical fiber. The light detector 22 to be used includes various devices such as a photomultiplier, photodiode, avalanche photodiode, and PIN photodiode. The light detector 22 to be selected only needs to have spectral sensitivity characteristics to allow detection of sufficient light intensity in the wavelength band of pulsed light P emitted from the pulsed light source 11. When the light to be detected is weak, a highly sensitive light detector or a high-gain light detector may be used.

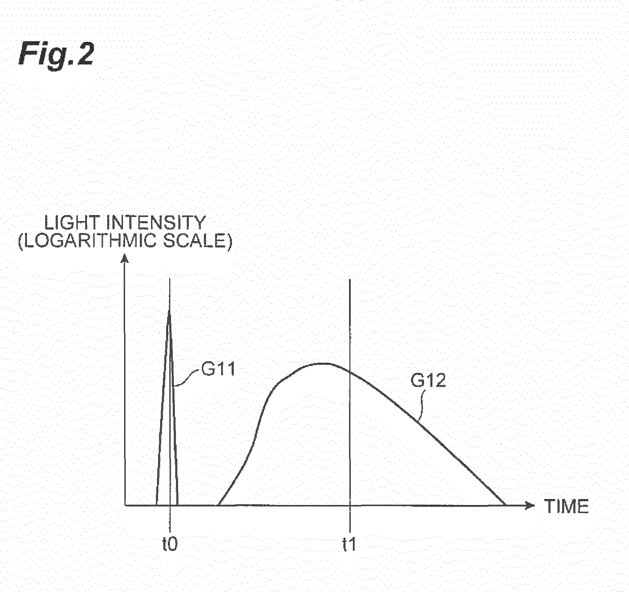

FIG. 2 is a graph illustrating an example of time variations of the light intensities of pulsed light P emitted from the light incident unit 10 and the detection light detected by the light detection unit 20. In FIG 2, the vertical axis denotes light intensity (logarithmic scale), and the horizontal axis denotes time. The graph G11 is a temporal profile (incident profile) of the pulsed light made incident on the measurement site B from the light incident unit 10 at time t₀. The graph G12 is a temporal profile (detection profile) having the detection light intensity corresponding to the incident pulsed light at time t₀. The time when the light propagated inside the measurement site B reaches the light detection position D is irregular due to its propagation conditions, and the propagated light decays due to scattering and absorption at the measurement site B. As illustrated by the graph G12 in FIG. 2, the detection profile thus becomes a fixed distribution curve.

Refer to FIG. 1 again. The estimation calculating unit 30A includes a temporal profile measuring unit 31 and an arithmetic processing unit 33A. The temporal profile measuring unit 31 is electrically connected with the light detector 22, and functions as signal processing means for performing a predetermined signal processing to the light detection signal from the light detector 22. The temporal profile measuring unit 31 acquires the temporal profile about the light intensity of the detection light based on the light detection signal from the light detector 22. For acquiring the temporal profile, the pulsed light source 11 provides a trigger signal indicating emission timing of the pulsed light P to the temporal profile measuring unit 31. Incidences and detections of pulsed light P performed at a plurality of measurement times yield a temporal profile at each of the measurement times.

The arithmetic processing unit 33A is arithmetic means for performing a predetermined calculation to the temporal profile acquired by the above signal processing means (temporal profile measuring unit 31). The arithmetic processing unit 33A includes a scattering coefficient calculating unit 33a and an estimating unit 33b. The scattering coefficient calculating unit 33a calculates scattering coefficient µₛ' of the measurement site B based on the temporal profile acquired by the temporal profile measuring unit 31. Note that scattering coefficient µₛ' to be described below is a concept including reduced scattering coefficient. Scattering coefficient µₛ' is preferably obtained by using, for example, a diffusion equation.

The estimating unit 33b acquires scattering coefficient µₛ' of the measurement site B from the scattering coefficient calculating unit 33a, and estimates an MRI index based on the scattering coefficient µₛ'. Herein, the storage unit 40 is formed of, for example, storage means such as a non-volatile memory, and preliminarily stores data indicating a correlation between MRI index and scattering coefficient µₛ'. The estimating unit 33b estimates MRI index by using the correlation data stored in the storage unit 40.

Alternatively, the storage unit 40 may preliminarily store data indicating correlation among MRI index, subarachnoid space thickness, and scattering coefficient µₛ'. In this case, a numerical number concerning the subarachnoid space thickness of the measurement site B is input from the parameter input unit 50. The subarachnoid space thickness of the measurement site B is preferably obtained by, for example, ultrasonographic examination. Then, the estimating unit 33b estimates MRI index by using the correlation data stored in the storage unit 40 and the subarachnoid space thickness input from the parameter input unit 50.

The display 80 is connected to the main body 70. The display 80 provides MRI index to a measurer and an examinee by displaying the MRI index estimated by the estimating unit 33b of the arithmetic processing unit 33A.

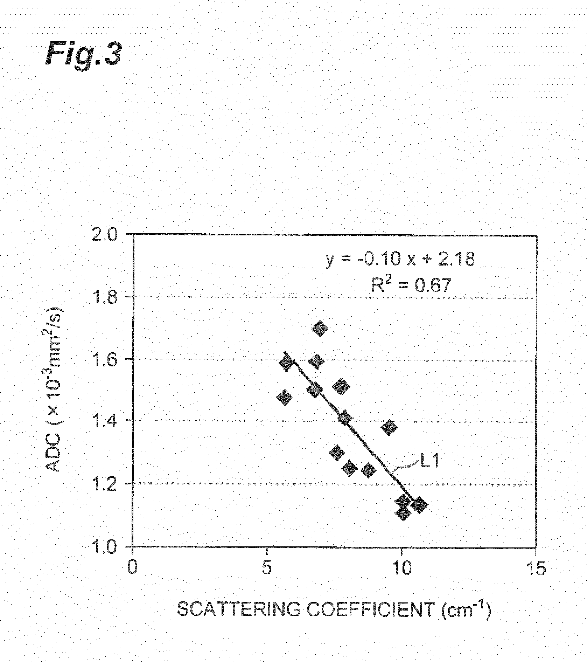

FIG. 3 is a graph illustrating a correlation between ADC and scattering coefficient µₛ' as an example of a correlation stored in the storage unit 40. In FIG. 3, the vertical axis denotes ADC (unit: × 10⁻³ mm²/sec), and the horizontal axis denotes scattering coefficient µₛ' (unit: cm⁻¹). Note that the graph indicates a case where subarachnoid space thickness is relatively small value such as not more than 2.5 mm. The correlation data between ADC and scattering coefficient µₛ' is preferably indicated by, for example, the approximate straight line L1 (y = -0.10x + 2.18: where, y denotes ADC and x denotes scattering coefficient µₛ', and the square of the correlation coefficient R between the two parameters is 0.67) illustrated in FIG 3.

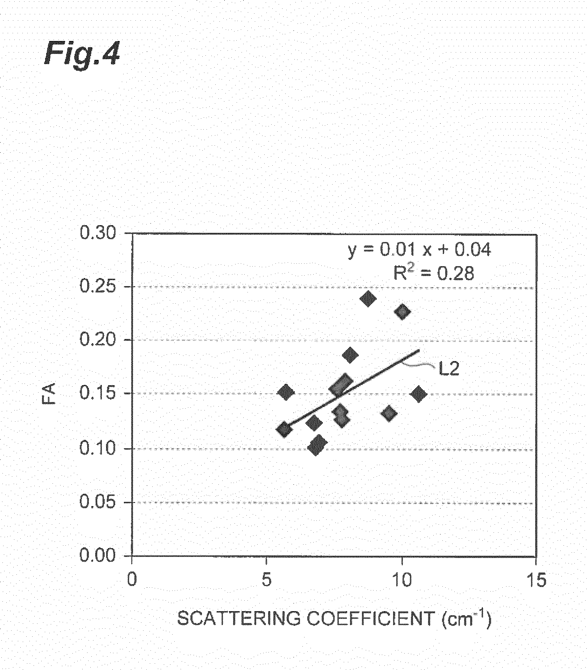

FIG. 4 is a graph illustrating a correlation between FA and scattering coefficient µₛ' as another example of the correlation stored in the storage unit 40. In FIG. 4, the vertical axis denotes FA (arbitrary unit) and the horizontal axis denotes scattering coefficient µₛ' (unit: cm⁻¹). Note that the graph indicates a case where subarachnoid space thickness is relatively small value such as not more than 2.5 mm. The correlation data between FA and scattering coefficient µₛ' is preferably indicated by, for example, the approximate straight line L2 (y = 0.01x + 0.04: where, y denotes FA and x denotes scattering coefficient µₛ', and the square of the correlation coefficient R between the two parameters is 0.28) illustrated in FIG. 4.

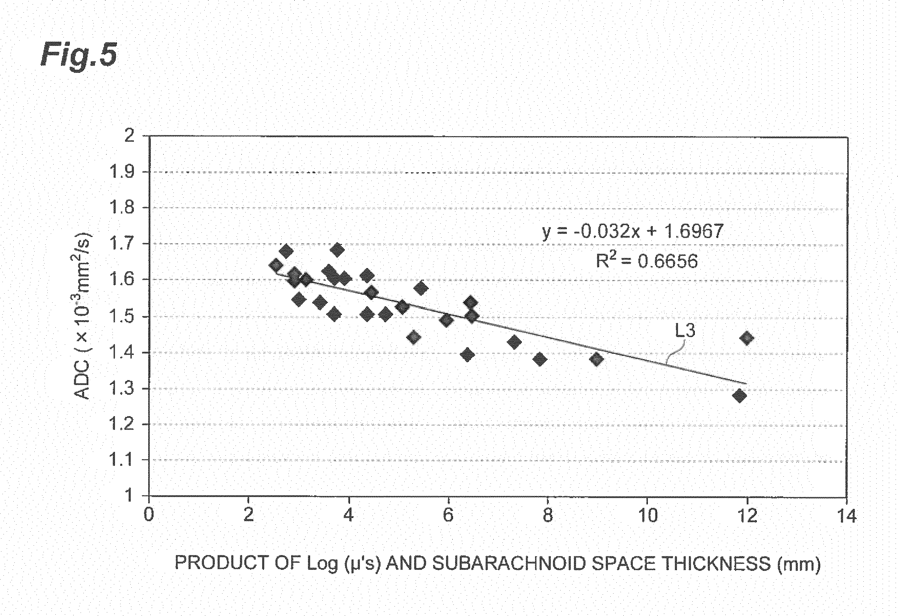

FIG. 5 is a graph illustrating a correlation between the product of the common logarithm value of scattering coefficient µₛ' and subarachnoid space thickness, and ADC as a still another example of the correlation stored in the storage unit 40. In FIG. 5, the vertical axis denotes ADC (unit: × 10⁻³mm²/sec), and the horizontal axis denotes the product of common logarithm value of scattering coefficient µₛ' (unit: cm⁻¹) and subarachnoid space thickness (unit: mm). The correlation data among ADC, subarachnoid space thickness, and scattering coefficient µₛ' is preferably indicated by, for example, the approximate straight line L3 (y = -0.032x + 1.6967: where, y denotes ADC, x denotes the product of the common logarithm value of scattering coefficient µₛ' and subarachnoid space thickness, and the square of the correlation coefficient R between the two parameters is 0.6656) illustrated in FIG 5.

Note that the correlation data stored in the storage unit 40 is not limited to the approximate straight lines L1 and L2, and the approximate straight line may be varied depending on increasing or decreasing of the number of data items.

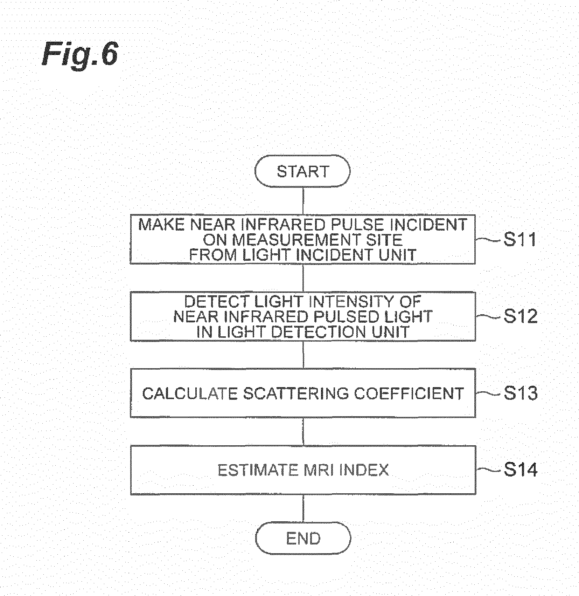

Operations of the biometric device 1A having the above configuration and an embodiment of the MRI index estimation method according to the invention will be described. FIG. 6 is a flowchart illustrating the operations of the biometric device 1A and the MRI index estimation method according to the embodiment. As shown in FIG. 6, first, near infrared pulsed light P is incident on the measurement site B from the light incident unit 10 (light incidence step S11). Next, the light detection unit 20 detects the light intensity of the near infrared pulsed light P propagated inside the measurement site B (light detection step S12).

Next, the scattering coefficient calculating unit 33a calculates scattering coefficient µₛ' of the measurement site B by time-resolved spectroscopy based on the result detected by the light detection unit 20 (scattering coefficient calculation step S13). Then, the estimating unit 33b estimates MRI index based on the correlation between scattering coefficient t µₛ' and MRI index (or the correlation among scattering coefficient µₛ' subarachnoid space thickness, and MRI index (MRI index estimation step S14).

Effects acquired by the biometric device 1A and the estimation method according to the embodiment having the above configuration will be described. A positron emission tomography (PET) device typically is expensive and large in size. In contrast, a near infrared spectroscopic measurement device such as the biometric device 1A can be inexpensive and small in size as compared with the PET device. The inventors have found through studies that there exists a significant correlation between scattering coefficient µₛ' of the measurement site B obtained by near-infrared spectroscopy and MRI index. The estimation method and the biometric device 1A according to the embodiment estimate MRI index based on scattering coefficient µₛ' of the measurement site B obtained by near-infrared spectroscopy. In this manner, estimating MRI index non-invasively by using near-infrared spectroscopy allows simple measurement at low cost as compared with the case of using an MRI device, making it possible to, for example, frequently monitor a cephalic part of a high-risk infant.

Moreover, as described above, when the estimating unit 33b estimates MRI index, MRI index may be estimated by using correlative relationship among scattering coefficient µₛ', subarachnoid space thickness, and MRI index. According to the studies by the present inventors, the relationship between scattering coefficient µₛ' and MRI index significantly changes depending on subarachnoid space thickness. Therefore, like the embodiment, estimating MRI index using the correlative relationship among scattering coefficient µₛ', subarachnoid space thickness, and MRI index allows estimation of MRI index more precisely.

Furthermore, like the embodiment, MRI index may be at least one of ADC and FA. As shown in FIGS. 3 to 5, the inventers have found through studies a very significant correlation between the MRI indexes and scattering coefficient µₛ' of the measurement site B.

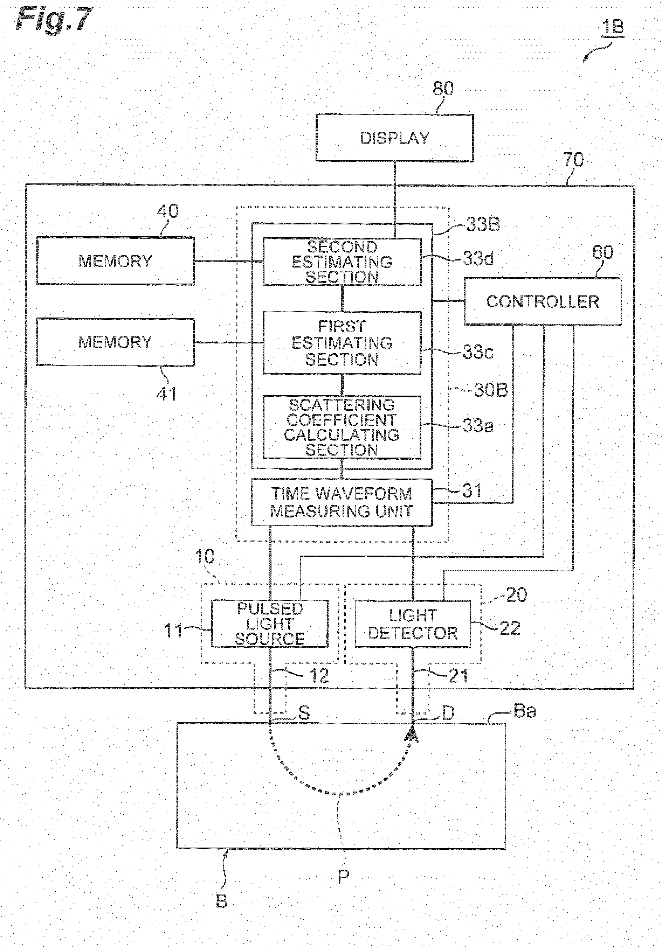

(Second Embodiment) FIG 7 is a block diagram schematically illustrating a configuration of a second embodiment of the biometric device according to the invention. The biometric device 1B is a device that estimates MRI index (MRI parameter) based on scattering coefficient of the measurement site B of a living body obtained by time-resolved spectroscopy using near-infrared light. The definitions of MRI index and scattering coefficient are same as those in the first embodiment. The biometric device 1B according to the embodiment includes an estimation calculating unit 30B instead of the estimation calculating unit 30A equipped in the biometric device 1A according to the first embodiment. The structure of the biometric device 1B other than the estimation calculating unit 30B is the same as the structure of the biometric device 1A according to the first embodiment.

The estimation calculating unit 30B includes a temporal profile measuring unit 31 and an arithmetic processing unit 33B. The structure of the temporal profile measuring unit 31 is same as that in the first embodiment. The arithmetic processing unit 33B is arithmetic means for performing a predetermined calculation to the temporal profile acquired by the temporal profile measuring unit 31. The arithmetic processing unit 33B includes a scattering coefficient calculating unit 33a, a first estimating unit 33c, and a second estimating unit 33d. The scattering coefficient calculating unit 33a calculates scattering coefficient µₛ' of the measurement site B based on the temporal profile acquired by the temporal profile measuring unit 31.

The first estimating unit 33c acquires scattering coefficient µₛ' of the measurement site B from the scattering coefficient calculating unit 33a, and estimates the subarachnoid space thickness of the measurement site B based on the scattering coefficient µₛ'. Herein, a storage unit 41 is formed of storage means, for example, such as a non-volatile memory, and preliminarily stores the data indicating correlation between scattering coefficient µₛ' and subarachnoid space thickness. The first estimating unit 33c estimates subarachnoid space thickness by using the correlation data stored in the storage unit 41.

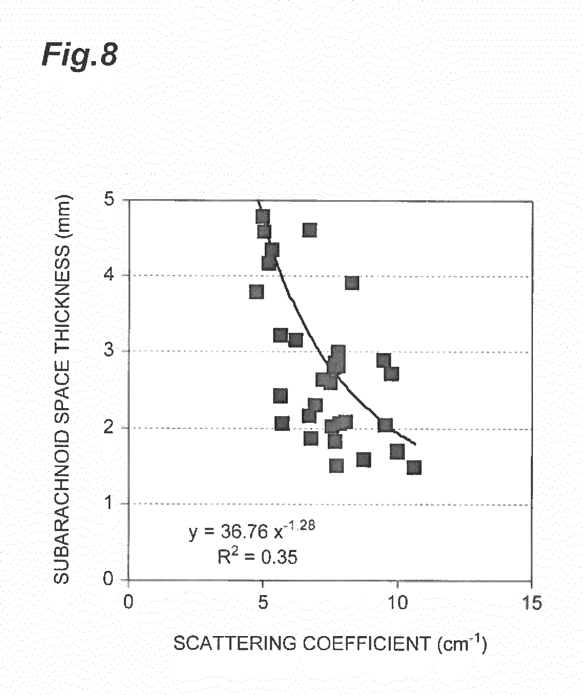

FIG. 8 is a graph illustrating a correlation between subarachnoid space thickness and scattering coefficient µₛ' as an example of a correlation stored in the storage unit 41. In FIG. 8, the vertical axis denotes subarachnoid space thickness (unit: mm), and the horizontal axis denotes scattering coefficient µₛ' (unit: cm⁻¹). The correlation data between subarachnoid space thickness and scattering coefficient µₛ' is preferably indicated by, for example, the approximate curve L4 (y = 36.76x^{-1,28} : where, y denotes subarachnoid space thickness, x denotes scattering coefficient µₛ', and the square of the correlation coefficient R between the two parameters is 0.35) illustrated in FIG. 8.

The second estimating unit 33d acquires scattering coefficient µₛ' of the measurement site B from the scattering coefficient calculating unit 33a, and acquires the estimation value of the subarachnoid space thickness of the measurement site B from the first estimating unit 33c. The second estimating unit 33d estimates MRI index based on the scattering coefficient µₛ and the subarachnoid space thickness estimation value. Herein, the storage unit 40 is formed of, for example, storage means such as a non-volatile memory, and preliminarily stores data indicating the correlation among MRI index, subarachnoid space thickness, and scattering coefficient µₛ'. The second estimating unit 33d estimates MRI index by using the correlation data stored in the storage unit 40.

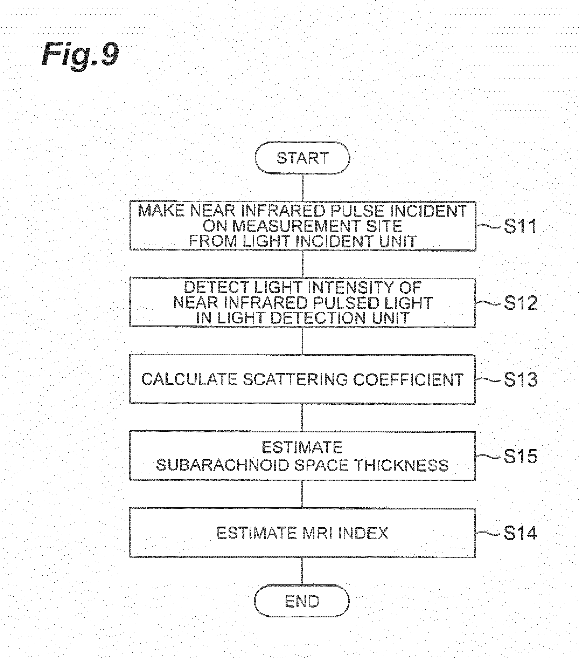

FIG. 9 is a flowchart illustrating operations of the biometric device 1B and an MRI index estimation method according to the embodiment. As shown in FIG 9, light incidence step S11, light detection step S12, and scattering coefficient calculation step S13 are performed similar to the first embodiment (see FIG. 6). Then, in the embodiment, the first estimating unit 33c estimates subarachnoid space thickness from the correlation between scattering coefficient t µₛ' and subarachnoid space thickness (subarachnoid space thickness estimation step S15). Then, the second estimating unit 33d estimates MRI index from the correlation among scattering coefficient µₛ', subarachnoid space thickness, and MRI index (MRI index estimation step S14).

Similar to the first embodiment, the biometric device 1B and the estimation method according to the embodiment having the above configuration allow simple measurement with low cost as compared with the case of using an MRI device. Furthermore, estimation of the MRI index using the correlative relationship among scattering coefficient t µₛ', subarachnoid space thickness, and MRI index allows estimation of MRI index more precisely.

The inventors have found that there exists a significant correlation also between scattering coefficient µₛ' of the measurement site B obtained by near-infrared spectroscopy and subarachnoid space thickness. Estimating subarachnoid space thickness based on the scattering coefficient µₛ' of the measurement site B obtained by near-infrared spectroscopy like the estimation method and the biometric device 1B according to the embodiment eliminates the need to separately measure subarachnoid space thickness by an ultrasonographic examination or the like, allowing more simple measurement.

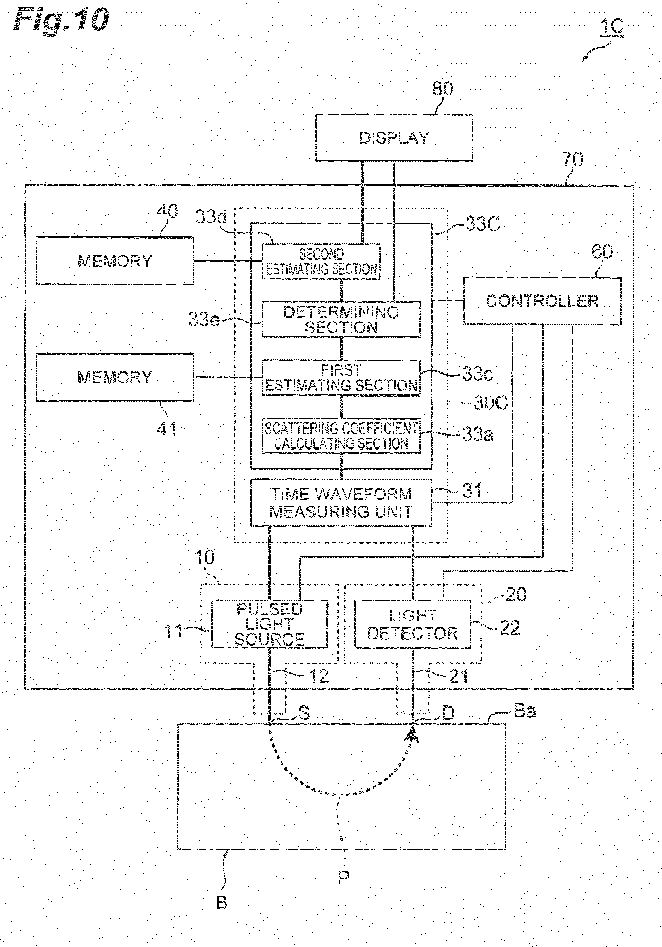

(Third Embodiment) FIG 10 is a diagram schematically illustrating a configuration of a biometric device 1C as a third embodiment of the biometric device according to the invention. The biometric device 1C according to the modification includes an estimation calculating unit 30C instead of the estimation calculating unit 30B equipped in the biometric device 1B according to the second embodiment. The estimation calculating unit 30C includes a temporal profile measuring unit 31 and an arithmetic processing unit 33C. The structure of the temporal profile measuring unit 31 is the same as that in the first embodiment.

The arithmetic processing unit 33C further includes a determining unit 33e in addition to the scattering coefficient calculating unit 33a, the first estimating unit 33c, and the second estimating unit 33d equipped in the arithmetic processing unit 33C of the second embodiment. The determining unit 33e acquires an estimation value of subarachnoid space thickness of the measurement site B from the first estimating unit 33c to compare the subarachnoid space thickness estimation value with a predetermined threshold value. The determining unit 33e causes the display 80 to display "measurement impossible" when the subarachnoid space thickness estimation value is larger than the predetermined threshold value (or not less than the predetermined threshold value). In this case, estimation calculating processing in the arithmetic processing unit 33C is suspended. Alternatively, when the determining unit 33e determines that the subarachnoid space thickness estimation value is smaller than the predetermined threshold value (or not more than the predetermined threshold value), the second estimating unit 33d estimates MRI index based on the correlation between scattering coefficient µₛ' and the MRI index (for example, see FIGS. 3 and 4). The predetermined threshold value is arbitrarily determined, and is 2.5 mm in an example.

Similar to the first embodiment, the biometric device 1C and the estimation method according to the modification allows simple measurement with low cost as compared with the case of using an MRI device. Moreover, limiting the measurement to the case where subarachnoid space thickness is smaller than a predetermined threshold suppresses influence on the estimation value due to the subarachnoid space thickness, allowing estimation of MRI index more precisely. To suppress subarachnoid space thickness to be smaller than a predetermined threshold, for example, it is preferable to measure occipital region in an upward facing state or temporal region on the lower side in a laterally facing state. Note that, in the embodiment, the first estimating unit 33c acquires subarachnoid space thickness to be input to the determining unit 33e by estimation calculation, but a numerical value concerning subarachnoid space thickness may be input from the parameter input unit similar to the first embodiment.

The MRI index estimation method and the biometric device according to the invention are not limited to the above embodiments, and various modifications can be made thereto. For example, although each of the embodiment estimates MRI index based on scattering coefficient, MRI index may be estimated based on various parameters having a correlation with scattering coefficient, for example, such as average light path length. In this case, it is sufficient that the storage unit stores the data indicating correlation between the parameter and MRI index instead of the correlative relationships illustrated in the above FIGS. 3 to 5, and the estimation calculating unit estimate MRI index using the correlation data. Alternatively, the estimation calculating unit may estimate MRI index by using correlation data indicating the correlation among the parameter, subarachnoid space thickness, and MRI index stored in the storage unit.

In the above embodiments, the case where the invention is applied to time-resolved spectroscopy, but the invention can be also applied to another method capable of measuring scattering coefficient or a parameter having a correlation with scattering coefficient (for example, phase modulation spectroscopy). Moreover, the equation (arithmetic equation) used for estimating MRI index is not limited to the straight lines as illustrated in FIGS. 3 to 5, and various relational expressions can apply to the estimation. Note that the storage unit 40 is capable of preliminarily storing the relationships between a plurality of scattering coefficients µₛ' and MRI indexes corresponding to the respective scattering coefficients µₛ' as a table or an arithmetic equation. Moreover, in the above embodiments, ADP and FA are exemplified as MRI index to be estimated, but the MRI index to be estimated by the invention is not limited thereto.

In the above embodiments, cephalic part is exemplified as the measurement site, and MRI index is estimated with high accuracy by using the correlation among scattering coefficient, subarachnoid space thickness, and the MRI index. However, MRI index may be estimated by using correlation among scattering coefficient, thickness of skull, and MRI index. Alternatively, when the measurement site is other than cephalic part (for example, muscle or abdominal area), MRI index may be estimated by using correlation among scattering coefficient, thickness of fat layer, and MRI index. In this manner, using correlation with various multi-layered organizations other than subarachnoid space thickness also allows estimation of MRI index with high accuracy.

The above embodiments exemplify the case where the number of each of the light incident position S and the light detection position D is one (one point incidence one point detection), but multipoint measurement is also possible (one point incidence multipoint detection, multipoint incidence one point detection, or multipoint incidence multipoint detection). Acquiring the information concerning a plurality of different depths allows separation of information depending on the depths, making it possible to improve accuracy of calculating scattering coefficient. Moreover, for an infant, measurement on anterior fontanel where no bone exists allows the information in the brain to be more readily included in the scattering coefficient, resulting in more significant correlation with MRI index.

Note that the above first to third embodiments are summarized as follows.

### (1) Output of Pulsed Light (pulsed light source 11)

When a trigger signal is output from the controller 60, and pulsed light P is output from the pulsed light source 11 to the light incident position S of the measurement site B in synchronization with the trigger signal, the light incident on the measurement site B is passed through inside of the measurement site B and is output to the outside from the light detection position D.

### (2) Detection of Light Passed through Organization (light detector 22)

The light detector 22 detects the pulsed light P output from the light detection position D and inputs a detection signal to the temporal profile measuring unit 31. The width of the detection signal corresponding to the pulsed light is spread in terms of time as illustrated in FIG. 2. A trigger signal indicating emission timing of the pulsed light P is also input to the temporal profile measuring unit 31.

### (3) Calculation of Scattering Coefficient Based on Detected Light (scattering coefficient calculating unit 33a)

When the light of near-infrared wavelength band (760 nm to 1400 nm) is irradiated on body tissue, the light scatters in the tissue and absorption occurs in the tissue due to heme such as hemoglobin, or the like. The scattering coefficient calculating unit 33a is capable of obtaining scattering coefficient µₛ' by using a time-resolved measurement method for scattering light. The time-resolved measurement method allows calculation by fitting the observed time response data (G12) shown in FIG. 2 to the theoretical output signal intensity T(t) = A x t^{-5/2} × exp(-µₐct)exp(-ρ²/(4D_{f}ct)) = A × t^{-5/2} × exp (-µₐct)exp[-ρ²/{4/(3µₛ') × ct}] obtained by a light diffusion equation. That is, the observed signal intensity (G12) output from the light detector and the theoretical T(t) are compared to determine scattering coefficient µₛ' by least squares method such that the sum of the square of the difference between the observed data and the theoretical data in each time becomes minimum. Note that A is proportional constant, t is elapsed time from when temporal response profile starts rising, µₐ is absorption coefficient, µₛ' is equivalent scattering coefficient (reduced scattering coefficient), D_{f} is optical diffusion coefficient (D_{f} = 1/(3µₛ')), c is velocity of light in the tissue, ρ is the distance from the light incident position S to the light detection position D. Note that the equation used for the fitting may be the one using various adjustments, or the correlated function between peak position of temporal response profile and scattering coefficient µₛ' may be calculated to calculate the scattering coefficient µₛ' from the peak position. Note that these coefficients other than the target scattering coefficient µₛ' are preliminarily obtained and given in the operation.

(4) Reading out of MRI Index Corresponding to Scattering Coefficient (estimating unit 33b) A table indicating correlation between scattering coefficient µₛ' and MRI index Z is stored in the storage unit 40. When the value of the calculated scattering coefficient µₛ' is input to the table (scattering coefficient µₛ'; MRI index Z) of the database, the value of the MRI index Z corresponding to the value is read out, and the read out value is regarded as the estimation value.

As described above, the table (scattering coefficient µₛ'; MRI index Z) is stored in the table of the first embodiment. As a modification, a table using three parameters (scattering coefficient µₛ'; MRI index Z; subarachnoid space thickness K) can be used, and the MRI index Z corresponding to the parameters can be read out by inputting the values of scattering coefficient µₛ' and subarachnoid space thickness K to the table.

In the first embodiment, subarachnoid space thickness K is manually input to the estimating unit 33b via the parameter input unit 50 (FIG. 1), but in the second embodiment, the value of subarachnoid space thickness K corresponding to scattering coefficient µₛ' is estimated based on scattering coefficient µₛ' and is input to the second estimating unit 33d (FIG. 7). In the third embodiment, whether the value of subarachnoid space thickness K is a thin value enough to obtain MRI index Z is determined with reference to a threshold (determining unit 33e), and MRI index Z is obtained for the one determined as good result.

As described above, the above biometric device includes the pulsed light source 11, the light detector 22 for detecting pulsed light output from the pulsed light source 11 and passed through a measurement site, the scattering coefficient calculating unit 33a for obtaining, based on a temporal response profile output from the light detector 22, a scattering coefficient of the measurement site corresponding to the temporal response profile, the storage unit 40 for preliminarily storing, as a table or an arithmetic equation, relationships between a plurality of scattering coefficients µₛ' and MRI indexes Z corresponding to the respective scattering coefficients µₛ', and the estimating unit for inputting a value of the scattering coefficient µₛ' output from the scattering coefficient calculating unit 33a to the table or the arithmetic equation in the storage unit 40 to obtain one of the MRI indexes Z corresponding to the input scattering coefficient µₛ'.

The storage unit 40 also preliminarily stores, as a table or an arithmetic equation, relationships among parameters and subarachnoid space thicknesses, the parameters being the scattering coefficients µₛ' and the MRI indexes Z, in addition to the scattering coefficients µₛ' and the MRI indexes Z.

Although the MRI index Z is the above ADC and/or FA, the relationships between the parameters and scattering coefficient µₛ' can be preliminarily obtained by using an MRI device. Furthermore, the above controlling is executed by a computer, and can be executed by the controller 60 and/or a program stored in the estimation calculating unit.

Note that the scattering coefficient may be obtained by a phase-contrast method. In this case, a light source that output intensity modulated light may be used instead of the pulsed light source 11.

### Reference Signs List

- 1A, 1B, 1C: biometric device
- 10: light incident unit
- 11: pulsed light source
- 12: light guide for light incidence
- 20: light detection unit
- 21: light guide for light detection
- 22: light detector
- 30A, 30B, 30C: estimation calculating unit
- 31: temporal profile measuring unit
- 33A, 33B, 33C: arithmetic processing unit
- 33a: scattering coefficient calculating unit
- 33b: estimating unit
- 33c: first estimating unit
- 33d: second estimating unit
- 33e: determining unit
- 40, 41: storage unit
- 50: parameter input unit
- 60: controller
- 70: main body
- 80: display
- B: measurement site
- D: light detection position
- L1 to L3: approximate straight line
- L4: approximate curve
- P: near infrared pulsed light
- S: light incident position
- D: light detection position

## Claims

1. An MRI index estimation method **characterized by** comprising estimating an MRI index based on a scattering coefficient (µₛ') of a measurement site (B) or a parameter having a correlation with the scattering coefficient (µₛ'), the scattering coefficient (µₛ') being obtained by a near-infrared spectroscopy based on a detection result of near-infrared light made incident on the measurement site (B) and propagated inside the measurement site (B).

2. The MRI index estimation method according to claim 1, wherein the MRI index is estimated by using a correlative relationship among the scattering coefficient (µₛ') or the parameter, a subarachnoid space thickness, and the MRI index when the MRI index is estimated.

3. The MRI index estimation method according to claim 1 or 2, wherein the MRI index is at least one of an apparent diffusion coefficient (ADC) and a fractional anisotopy (FA).

4. A biometric device comprising:
a light incident unit (10) for making near-infrared light incident on a measurement site (B);
a light detection unit (20) for detecting the near-infrared light propagated inside the measurement site (B); and
**characterized by**
an estimation calculating unit (30A, 30B, 30C) for obtaining a scattering coefficient (µₛ') of the measurement site (B) or a parameter having a correlation with the scattering coefficient (µₛ') by a near-infrared spectroscopy based on a detection result in the light detection unit (20), and estimating an MRI index based on the scattering coefficient (µₛ') or the parameter.

5. The biometric device according to claim 4, further comprising a first storage unit (40) for storing a correlative relationship among the scattering coefficient (µₛ') or the parameter, a subarachnoid space thickness, and the MRI index, wherein
the estimation calculating unit (30A, 30B, 30C) is configured to estimate the MRI index by using the correlative relationship.

6. The biometric device according to claim 4 or 5, wherein the MRI index is at least one of an apparent diffusion coefficient (ADC) and a fractional anisotopy (FA).

7. The biometric device according to any one of claims 4 to 6, wherein,
the light incident unit (10) includes a pulsed light source (11) for generating a pulsed light (P) as the near infrared light;
the light detection unit (20) includes a light detector (22) for detecting the near infrared pulsed light (P) output from the pulsed light source (11) and passed through the measurement site (B);
the estimation calculating unit (30A, 30B, 30C) includes a scattering coefficient calculating unit (33a) for obtaining, based on a temporal response profile output from the light detector (22), the scattering coefficient (µₛ') of the measurement site (B) corresponding to the temporal response profile;
a second storage unit (41) for preliminarily storing, as a table or an arithmetic equation, relationships between a plurality of scattering coefficients and MRI indexes corresponding to the respective scattering coefficients; and
the estimation calculating unit (30A, 30B, 30C) further includes an estimating unit (33b) for inputting a value of the scattering coefficient (µₛ') output from the scattering coefficient calculating unit (33a) to the table or the arithmetic equation in the second storage unit (41) to obtain one of the MRI indexes corresponding to the input scattering coefficient.

8. The biometric device according to claim 7, wherein the second storage unit (41) is configured to preliminarily store, as a table or an arithmetic equation, relationships among parameters and subarachnoid space thicknesses, the parameters being the scattering coefficients and the MRI indexes, in addition to the scattering coefficients and the MRI indexes.

## Patentansprüche

1. MRI-Indexschätzverfahren, **gekennzeichnet durch**
Schätzen eines MRI-Index auf der Grundlage eines Streukoeffizienten (µ_{S}') einer Messstelle (B) oder eines Parameters mit einer Korrelation zu dem Streukoeffizienten (µ_{S}'), wobei der Streukoeffizient (µ_{S}') durch Spektroskopie im nahen Infrarotbereich auf der Grundlage eines Detektionsergebnisses von Licht im nahen Infrarotbereich erhalten wird, das auf die Messstelle (B) gelenkt wird und sich im Inneren der Messstelle (B) ausbreitet.

2. MRI-Indexschätzverfahren nach Anspruch 1, wobei der MRI-Index unter Verwendung einer korrelativen Beziehung zwischen dem Streukoeffizienten (µ_{S}') oder dem Parameter, einer Dicke eines subarachnoiden Raums und dem MRI-Index erhalten wird, wenn der MRI-Index geschätzt wird.

3. MRI-Indexschätzverfahren nach Anspruch 1 oder 2, wobei der MRI-Index ein Koeffizient für offensichtliche Diffussion (ADC) und/oder eine fraktionelle Anisotropie (FA) ist.

4. Biometrische Vorrichtung, mit:
einer Lichteinfallseinheit (10), die ausgebildet ist, Licht im nahen Infrarotbereich auf eine Messstelle (B) einfallen zu lassen;
einer Lichtdetektionseinheit (20) zum Detektieren des Lichts im nahen Infrarotbereich, das sich im Inneren der Messstelle (B) ausbreitet;
**gekennzeichnet durch**
eine Schätzungsberechnungseinheit (30A, 30B, 30C) zum Erhalten eines Streukoeffizienten (µ_{S}') der Messstelle (B) oder eines Parameters mit einer Korrelation zu dem Streukoeffizienten (µ_{S}') durch Spektroskopie im nahen Infrarotbereich auf der Grundlage eines Detektionsergebnisses in der Lichtdetektionseinheit (20), und zum Abschätzen eines MRI-Index auf der Grundlage des Streukoeffizienten (µ_{S}') oder des Parameters.

5. Biometrische Vorrichtung nach Anspruch 4, die ferner eine erste Speichereinheit (40) zum Speichern einer korrelativen Beziehung zwischen dem Streukoeffizienten (µ_{S}') oder dem Parameter, einer Dicke eines subarachnoiden Raums und dem MRI-Index aufweist, wobei die Schätzungsberechnungseinheit (30A, 30B, 30C) ausgebildet ist, den MRI-Index unter Anwendung der korrelativen Beziehung zu schätzen.

6. Biometrische Vorrichtung nach Anspruch 4 oder 5, wobei der MRI-Index ein Koeffizient für offensichtliche Diffusion (ADC) und/oder eine fraktionelle Anisotropie (FA) ist.

7. Biometrische Vorrichtung nach einem der Ansprüche 4 bis 6, wobei
die Lichteinfallseinheit (10) eine gepulste Lichtquelle (11) zur Erzeugung von gepulstem Licht (P) als das Licht im nahen Infrarotbereich aufweist;
die Lichtdetektionseinheit (20) einen Lichtdetektor (22) zum Erfassen des gepulsten Lichts im nahen Infrarotbereich (P) aufweist, das aus der gepulsten Lichtquelle (11) ausgegeben wird und die Messstelle (B) durchläuft;
die Schätzungsberechnungseinheit (30A, 30B, 30C) eine Streukoeffizientenberechnungseinheit (33a) aufweist, die auf der Grundlage eines aus dem Lichtdetektor (22) ausgegebenen zeitlichen Antwortprofils den Streukoeffizienten (µ_{S}') der Messstelle (B) entsprechend dem zeitlichen Antwortprofil ermittelt;
eine zweite Speichereinheit (41) vorgesehen ist, die als eine Tabelle oder eine arithmetische Gleichung vorläufig Beziehungen zwischen mehreren Streukoeffizienten und MRI-Indizes, die den jeweiligen Streukoeffizienten zugeordnet sind, speichert; und
die Abschätzungsberechnungseinheit (30A, 30B, 30C) ferner eine Schätzeinheit (33b) zum Eingeben eines Wertes des Streukoeffizienten (µ_{S}'), der von der Streukoeffizientenberechnungseinheit (33a) ausgegeben wird, in die Tabelle oder die arithmetische Gleichung in der zweiten Speichereinheit (41) aufweist, um einen der MRI-Indizes zu erhalten, der dem eingegebenen Streukoeffizienten entspricht.

8. Biometrische Vorrichtung nach Anspruch 7, wobei die zweite Speichereinheit (41) ausgebildet ist, zusätzlich zu den Streukoeffizienten und den MRI-Indizes Beziehungen zwischen Parametern und Dicken von subarachnoiden Räumen vorläufig als eine Tabelle oder eine arithmetische Gleichung zu speichern, wobei die Parameter die Streukoeffizienten und die MRI-Indizes sind.

## Revendications

1. Procédé d'estimation d'indice MRI **caractérisé en ce qu'il** comprend l'estimation d'un indice MRI sur la base d'un coefficient de diffusion (µₛ') d'un site de mesure (B) ou d'un paramètre ayant une corrélation avec le coefficient de diffusion (µₛ'), le coefficient de diffusion (µₛ') étant obtenu par une spectroscopie proche infrarouge sur la base d'un résultat de détection d'une lumière proche infrarouge rendue incidente sur le site de mesure (B) et propagée à l'intérieur du site de mesure (B).

2. Procédé d'estimation d'indice MRI selon la revendication 1, l'indice MRI étant estimé en utilisant une relation de corrélation parmi le coefficient de diffusion (µₛ') ou le paramètre, une épaisseur de l'espace sous-arachnoïdien, et l'indice MRI lorsque l'indice MRI est estimé.

3. Procédé d'estimation d'indice MRI selon la revendication 1 ou 2, l'indice MRI étant au moins l'un d'un coefficient de diffusion apparent (ADC) et d'une anisotropie fractionnée (FA).

4. Dispositif biométrique comprenant :
une unité incidente de lumière (10) pour rendre la lumière proche infrarouge incidente sur un site de mesure (B) ;
une unité de détection de lumière (20) pour détecter la lumière proche infrarouge propagée à l'intérieur du site de mesure (B) ; et
**caractérisé par**
une unité de calcul d'estimation (30A, 30B, 30C) pour obtenir un coefficient de diffusion (µₛ') du site de mesure (B) ou un paramètre ayant une corrélation avec le coefficient de diffusion (µₛ') par une spectroscopie proche infrarouge sur la base d'un résultat de détection dans l'unité de détection de lumière (20), et l'estimation d'un indice MRI sur la base du coefficient de diffusion (µₛ') ou du paramètre.

5. Dispositif biométrique selon la revendication 4, comprenant en outre une première unité de stockage (40) pour stocker une relation de corrélation parmi le coefficient de diffusion (µₛ') ou le paramètre, une épaisseur de l'espace sous-arachnoïdien, et l'indice MRI,
l'unité de calcul d'estimation (30A, 30B, 30C) étant configurée pour estimer l'indice MRI en utilisant la relation de corrélation.

6. Dispositif biométrique selon la revendication 4 ou 5, l'indice MRI étant au moins l'un d'un coefficient de diffusion apparent (ADC) et d'une anisotropie fractionnée (FA).

7. Dispositif biométrique selon l'une quelconque des revendications 4 à 6,
l'unité incidente de lumière (10) comprenant une source de lumière pulsée (11) pour générer une lumière pulsée (P) comme la lumière proche infrarouge ;
l'unité de détection de lumière (20) comprenant un détecteur de lumière (22) pour détecter la lumière pulsée proche infrarouge (P) émise depuis la source de lumière pulsée (11) et passée à travers le site de mesure (B) ;
l'unité de calcul d'estimation (30A, 30B, 30C) comprenant une unité de calcul de coefficient de diffusion (33a) pour obtenir, sur la base d'un profil de réponse temporel émis depuis le détecteur de lumière (22), le coefficient de diffusion (µₛ') du site de mesure (B) correspondant au profil de réponse temporel ;
une seconde unité de stockage (41) pour stocker de manière préliminaire, sous la forme d'un tableau ou d'une équation arithmétique, les relations entre une pluralité de coefficients de diffusion et d'indices MRI correspondant aux coefficients de diffusion respectifs ; et
l'unité de calcul d'estimation (30A, 30B, 30C) comprenant en outre une unité d'estimation (33b) pour saisir une valeur du coefficient de diffusion (µₛ') émise depuis l'unité de calcul de coefficient de diffusion (33a) vers le tableau ou l'équation arithmétique dans la seconde unité de stockage (41) pour obtenir l'un des indices MRI correspondant au coefficient de diffusion saisi.

8. Dispositif biométrique selon la revendication 7, la seconde unité de stockage (41) étant configurée pour stocker de manière préliminaire, sous la forme d'un tableau ou d'une équation arithmétique, les relations parmi les paramètres et les épaisseurs de l'espace sous-arachnoïdien, les paramètres étant les coefficients de diffusion et les indices MRI, en plus des coefficients de diffusion et des indices MRI.
